# DEMANDE DE BREVET EUROPEEN

(11) **EP 1 130 392 A1**
(43) Date de publication de la demande: **05.09.2001**
(21) Numéro de dépôt: 01400439.4
(22) Date de dépôt: 20.02.2001
(51) Int. Cl.: G01N 33/10

(54) **Méthode de sélection d'amidons**

(30) Priorité: 02.03.2000 FR 0002810
(71) Demandeur: INSTITUT FRANCAIS DU PETROLE, 92852 Rueil-Malmaison Cedex (FR)
(72) Inventeur: Dubois, Isabelle, 7210 Avon (FR); Rousseau, Lionel, 78440 Issou (FR); Audibert Hayet, Annie, 78290 Croissy sur Seine (FR)

(57) **Abrégé**

Pour définir un échantillon d'amidon inconnu, alkylé ou non, réticulé ou non, et le classer dans une des trois zones I, II, et III définies par rapport au processus de gélatinisation et parmi lesquelles:
- la zone I correspond au gonflement du grain d'amidon natif lorsque la température augmente, ce gonflement s'accompagnant d'une augmentation de la viscosité apparente de la dispersion;
- la zone II est définie entre la température de gélatinisation et une température de l'ordre de 100°C, dans laquelle le grain d'amidon natif éclate et libère une partie des chaînes macromoléculaires; et
- la zone III est définie au-delà de 100°C et correspond à une système où les macromolécules qui constituent le grain d'amidon sont complètement solubilisées;
on met en oeuvre une méthode qui comprend:
- une première étape dans laquelle on centrifuge ledit échantillon préparé sous la forme d'une dispersion à faible concentration en amidon dont on peut déterminer la capacité de gonflement et située dans la zone I ou dans la zone II;
- une deuxième étape dans laquelle on détermine le diamètre moyen des particules d'amidon; et
- une troisième étape dans laquelle on réalise le vieillissement thermique de l'échantillon et on effectue de nouveau sur l'échantillon après vieilissement les déterminations des deux première étapes.

## Description

La présente invention a pour objet une méthode de sélection des amidons qui permet de définir un échantillon d'amidon inconnu, alkylé ou non, réticulé ou non, et de le classer dans une zone définie en relation avec le processus de gélatinisation.

Les amidons sont des polymères naturels issus de la biomasse. A l'état natif, les amidons se présentent sous forme granulaire. Ils peuvent être de différentes origines (par exemple céréales, tubercules ou légumineuses) et le diamètre des grains peut atteindre 100 µm. Ils sont constitués de deux chaînes macromoléculaires, l'amylose et l'amylopectine. Ces macromolécules sont composées d'unités anhydroglucose reliées en majorité par des liaisons α-1,4 et quelques liaisons α-1,6. La proportion entre l'amylose et l'amylopectine est variable et est fonction de l'espèce considérée. Elle varie entre 1 et 35 % en masse pour l'amylose et entre 99 et 65 % en masse pour l'amylopectine.

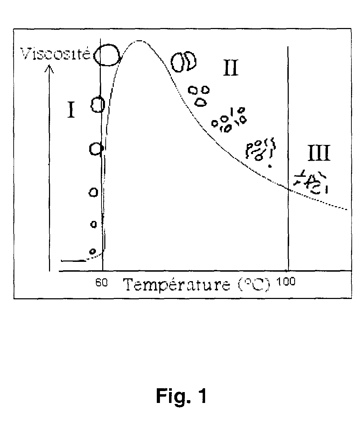

A l'état natif, les amidons sont insolubles à froid. Les propriétés physico-chimiques des grains d'amidons sont gouvernées par le processus de gélatinisation rapporté ci-dessous. La gélatinisation est un processus hydrothermique. Il est représenté sur la Figure 1 annexée par l'évolution de la viscosité apparente en fonction de la température.

Sur ce diagramme, on distingue trois zones, I, II, et III :
- La zone I correspond au gonflement du grain d'amidon natif lorsque la température augmente. Elle s'arrête à une température critique, dite "température de gélatinisation" comprise entre 50 °C et 70 °C. Ce gonflement s'accompagne d'une augmentation de la viscosité apparente de la dispersion.
- La zone II est définie entre la température de gélatinisation et une température de l'ordre de 100 °C. Dans cette zone, le grain d'amidon natif éclate et libère une partie des chaînes macromoléculaires dont il est constitué. Le système est alors formé de fragments de grains et de macromolécules en solution.
- La zone III, définie au-delà de 100 °C, correspond à un système où les macromolécules qui constituent le grain d'amidon sont complètement solubilisées.

La modification chimique du grain d'amidon peut permettre le déplacement de la température de gélatinisation et l'amélioration de la stabilité thermique ou mécanique de ces composés. Ainsi, les amidons peuvent être réticulés (par exemple par l'épichlorohydrine ou l'acide adipique) et/ou alkylés (par exemple hydroxyalkylés ou carboxyalkylés). Ces modifications chimiques peuvent avoir lieu dans les zones I, II ou III. Le degré de réticulation est compris entre 0,05 % et 1 %. Dans le cas des amidons alkylés, le degré de substitution par des chaînes alkyles, c'est-à-dire le nombre de chaînes alkyles pour 100 unités anhydroglucose, peut être d'au moins 0,5.

La méthode de l'invention comprend différentes étapes qui permettent de sélectionner un ou plusieurs amidons pour une application bien déterminée : pour les fluides de puits, en cosmétologie, dans les techniques d'encapsulation, en galénique, en pharmacie, dans l'agro-alimentaire, la papeterie, le traitement des eaux et des rejets, etc. Les échantillons sont définis par leurs propriétés physico-chimiques.

Dans une première étape, on centrifuge à une vitesse suffisante, par exemple d'environ 700 × g, une dispersion d'amidon préalablement préparée avec une faible concentration en polymère, par exemple de 1 à 10 g/l.
- L'absence de sédiment après centrifugation signifie que l'échantillon étudié se présente sous l'aspect d'une solution de macromolécules. L'échantillon est alors situé dans la zone III.
- La présence d'une phase sédimentée et d'une phase non sédimentée, signifie que l'échantillon se présente sous l'aspect d'une suspension dont la proportion de chacune des phases peut être déterminée. La phase sédimentée permet de déterminer la quantité d'eau absorbée pour 1 g de polymère en présence d'un excès d'eau, c'est la capacité de gonflement ; l'échantillon est alors situé dans la zone I ou dans la zone II.

Dans une deuxième étape, on détermine le diamètre moyen des particules d'amidon à une faible concentration en polymère. Cette mesure est réalisée à l'aide d'un granulomètre laser. Le diamètre ainsi mesuré est fonction de la zone où se trouve l'échantillon, mais également du degré de réticulation chimique. Ainsi, le diamètre moyen d'un échantillon situé dans la zone I est proche de celui du grain d'amidon natif, entre 20 µm et 60 µm selon l'origine de l'espèce. Le diamètre moyen d'un échantillon situé dans la zone II dépend du degré de réticulation chimique et est inférieur ou égal à celui d'un échantillon situé dans la zone I.

Au terme de ces analyses, dans une troisième étape, on étudie la stabilité thermique des amidons pour identifier leur appartenance à la zone I ou à la zone II.

Les conditions de cette étude thermique sont en général les suivantes : températures supérieures à 100 °C ; temps de vieillissement variable dans une cellule de test HP/HT (haute pression/haute température) ; contre-pression de 1 MPa ; préparation des dispersions dans l'eau désionisée ; viscosités des dispersions supérieures ou égales à 1 Pa.s (pour un cisaillement de 1 s⁻¹).

Après un vieillissement en température, la viscosité apparente de la dispersion est mesurée à 25 °C au moyen d'un rhéomètre CARRI-MED CSL 100® et comparée à celle obtenue avant le vieillissement thermique. Ensuite, on applique les deux premières étapes de la méthode décrites ci-dessus pour classer l'échantillon en zone I ou en zone II.
- L'impossibilité de déterminer le diamètre moyen des particules et la capacité de gonflement de l'échantillon après quelques heures de vieillissement thermique, avec une viscosité apparente encore significative, indique que le système se délite et que les macromolécules sont solubilisées ; on a alors affaire à un échantillon correspondant à la zone II.
- La possibilité de déterminer le diamètre moyen des particules et la capacité de gonflement après quelques heures de vieillissement, indique que des particules subsistent toujours dans la dispersion ; on a alors affaire à un échantillon correspondant à la zone I.

Par exemple dans l'application des amidons comme additifs pour fluides de puits, il est montré dans la demande de brevet ayant pour titre "Additif réducteur de filtrat et fluide de puits contenant cet additif", déposée le même jour, que le mélange d'un amidon alkylé et réticulé appartenant à la zone I (sous forme granulaire) ou II (sous forme fragmentée) avec un amidon natif ou alkylé appartenant à la zone III (sous forme de macromoléculaire hydrosoluble) présentait des propriétés avantageuses. Cette demande de brevet est incorporée dans la présente description du simple fait de sa mention.

Les exemples suivants illustrent l'invention sans la limiter.

### EXEMPLES

### Présentation des amidons :

On dispose de trois échantillons d'amidons inconnus, dérivés de l'amidon de pomme de terre. Ils sont notés référencés A, B et C. Ils peuvent avoir été hydroxyalkylés (par exemple hydroxypropylés) et/ou réticulés. La méthode de l'invention doit permettre de les classer dans les zones I, II ou III, définies par rapport au processus de gélatinisation.

### Propriétés physico-chimiques des amidons à 25 °C :

Les propriétés physico-chimiques correspondent aux deux premières étapes de la méthode de l'invention.

Les conditions opératoires sont les suivantes: concentration des échantillons: 3 g/litre ; solvant: eau désionisée ; vitesse de centrifugation : 700 × g ; température de centrifugation : 15 °C.

Les résultats sont présentés dans le Tableau 1.

**TABLEAU 1**

| **Référence de l'échantillon** | **Diamètre moyen (µm)** | **Capacité de gonflement (ml/g)** | **Phase non sédimentée (% masse)** |
|---|---|---|---|
| **A** | 64 | 12 | <20 |
| **B** | 60 | 17 | <20 |
| **C** | - | - | 100 |

A partir de ces données, la zone dans laquelle se situent les échantillons A et B ne peut pas être définie. La détermination du diamètre moyen des particules et de la capacité de gonflement montre que les échantillons A et B se situent soit dans la zone I, soit dans la zone II. Les 100 % de phase non sédimentée de l'échantillon C indiquent son appartenance à la zone III.

### Stabilité en température des dispersions d'amidon :

### Essai préliminaire :

Les conditions préliminaires sont : concentration des échantillons : 100 g/l; Solvant : eau désionisée ; température de 110 °C à 160 °C, avec un vieillissement de 16 heures ; contre-pression de 1MPa.

Les résultats sont présentés dans le Tableau 2.

**TABLEAU 2**

| **Référence de l'échantillon** | **Température limite de stabilité après vieillissement (°C)** |
|---|---|
| **A** | <160 |
| **B** | <140 |
| **C** | <120 |

Les échantillons A et B ont des températures limites de stabilité différentes, alors que leurs propriétés physico-chimiques sont du même ordre de grandeur à 25 °C. Des cinétiques de vieillissement sont alors réalisées sur ces deux derniers échantillons pour mieux les identifier. L'essai réalisé avec l'échantillon C, déjà identifié comme appartenant à la zone III et dont le résultat est donné à titre indicatif, montre que la stabilité thermique d'un système sous forme macromoléculaire est plus faible que celle d'un système appartenant à la zone I (amidon granulaire)ou à la zone II (amidon fragmenté).

### Essai sélectif :

Les conditions de l'essai sont : température de 160 °C pour l'échantillon A et de 140 °C pour l'échantillon B, avec un temps de vieillissement variable et une contre-pression de 1 MPa.

Les résultats sont présentés dans le Tableau 3.

**TABLEAU 3**

| **Référence de l'échantillon** | **A (160 °C)** | | | | **B (140 °C)** | | |
|---|---|---|---|---|---|---|---|
| **Temps (h)** | 0 | 1 | 2 | 3 | 0 | 3 | 6 |
| **Perte relative de viscosité apparente (%)** | 0 | 0 | 0 | 90 | 0 | 10 | 90 |
| **Diamètre moyen (µm)** | 64 | 105 | 110 | 60 | 60 | 69 | ― |
| **Capacité de gonflement (ml/g)** | 12 | 35 | 36 | 49 | 17 | 50 | ― |

La détermination du diamètre moyen et de la capacité de gonflement des particules à 25 °C est difficile pour l'échantillon B après 6 heures de vieillissement à 140°C. L'échantillon se délite sous agitation mécanique. Les mesures du diamètre moyen et de la capacité de gonflement de l'échantillon A sont possibles malgré une perte relative de la viscosité apparente de 90 %.

Selon la troisième étape de la méthode de l'invention, l'échantillon A est donc un échantillon correspondant à la zone I et l'échantillon B à la zone II.

## Revendications

1. Méthode pour définir un échantillon d'amidon inconnu, alkylé ou non, réticulé ou non, et le classer dans une des trois zones I, II, et III définies par rapport au processus de gélatinisation et parmi lesquelles :
- la zone I correspond au gonflement du grain d'amidon natif lorsque la température augmente, s'arrêtant à une température critique, dite température de gélatinisation, comprise entre 50 °C et 70 °C, ce gonflement s'accompagnant d'une augmentation de la viscosité apparente de la dispersion ;
- la zone II est définie entre la température de gélatinisation et une température de l'ordre de 100 °C, dans laquelle le grain d'amidon natif éclate et libère une partie des chaînes macromoléculaires dont il est constitué, le système étant alors formé de fragments de grains et de macromolécules en solution ; et
- la zone III est définie au-delà de 100 °C et correspond à un système où les macromolécules qui constituent le grain d'amidon sont complètement solubilisées ;
ladite méthode étant **caractérisée en ce qu**'elle comprend :
- une première étape dans laquelle on centrifuge ledit échantillon préparé sous la forme d'une dispersion à faible concentration en amidon dans l'eau à une vitesse suffisante, l'absence de sédiment après centrifugation indiquant que l'échantillon étudié se présente sous l'aspect d'une solution de macromolécules située dans la zone III ; et la présence d'une phase sédimentée et d'une phase non sédimentée indiquant que l'échantillon se présente sous l'aspect d'une suspension dont on peut déterminer la capacité de gonflement et située dans la zone I ou dans la zone II.
- une deuxième étape dans laquelle on détermine le diamètre moyen des particules d'amidon ; et
- une troisième étape dans laquelle on réalise le vieillissement thermique de l'échantillon et on effectue de nouveau sur l'échantillon après vieillissement les déterminations des deux première étapes, l'impossibilité de déterminer le diamètre moyen des particules et la capacité de gonflement de l'échantillon après quelques heures de vieillissement thermique, avec une viscosité apparente encore significative, indiquant que l'échantillon correspond à la zone II ; la possibilité de déterminer le diamètre moyen des particules et la capacité de gonflement après quelques heures de vieillissement indiquant que l'échantillon correspond à la zone I.

2. Méthode selon la revendication 1, **caractérisée en ce que** la dispersion d'amidon est préalablement préparée avec une concentration en polymère de 1 à 10 g/l, la préparation de la dispersion étant effectuée dans de l'eau désionisée.

3. Méthode selon la revendication 1 et 2 **caractérisée en ce que** dans la première étape on centrifuge à une vitesse d'environ 700 × g.

4. Méthode selon l'une des revendications 1 à 3 **caractérisée en ce que** dans la troisième étape le vieillissement thermique est réalisé dans une cellule de tests haute pression/haute température, à une température supérieure à 100 °C ; sous une contre-pression d'environ 1 MPa ; le vieillissement étant effectué dans de l'eau désionisée.

5. Méthode selon l'une des revendications 1 à 4 **caractérisée en ce que** l'on identifie un amidon de degré de réticulation compris entre 0,05 % et 1 %.

6. Méthode selon l'une des revendications 1 à 5 **caractérisée en ce que** l'on identifie un amidon alkylé de degré de substitution d'au moins 0,5.

7. Méthode selon l'une des revendications 1 à 6 **caractérisée en ce que** l'on évalue la température limite de stabilité d'un amidon, alkylé ou non, réticulé ou non.

8. Méthode selon l'une des revendications de 1 à 8 **caractérisée en ce qu**'on sélectionne un amidon pour une application déterminée, fluides de puits, cosmétologie, techniques d'encapsulation, galénique, pharmacie, agro-alimentaire, papeterie ou traitement des eaux et des rejets.
